(19) Europäisches Patentamt
European Patent Office
Office européen des brevets

(11) Veröffentlichungsnummer : **0 340 705 B1**

# (12) EUROPÄISCHE PATENTSCHRIFT

(45) Veröffentlichungstag der Patentschrift : **03.02.93 Patentblatt 93/05**

(51) Int. Cl.$^5$ : **C07C 317/12,** C08G 59/20, C08G 59/14, C08G 73/12, C08F 222/40, C08G 73/06

(21) Anmeldenummer : **89107880.0**

(22) Anmeldetag : **29.04.89**

## (54) Neues Polyphenol und daraus hergestellte Duromere.

(30) Priorität : **04.05.88 DE 3815050**

(43) Veröffentlichungstag der Anmeldung : **08.11.89 Patentblatt 89/45**

(45) Bekanntmachung des Hinweises auf die Patenterteilung : **03.02.93 Patentblatt 93/05**

(84) Benannte Vertragsstaaten : **CH DE FR GB IT LI NL**

(56) Entgegenhaltungen :
**DE-B- 1 259 880**
**US-A- 3 220 981**
**US-A- 3 419 624**

(73) Patentinhaber : **BASF Aktiengesellschaft**
**Carl-Bosch-Strasse 38**
**W-6700 Ludwigshafen (DE)**

(72) Erfinder : **Holoch, Jan, Dr.**
**Koenigsberger Strasse 22A**
**W-6906 Leimen (DE)**
Erfinder : **Peter, Roland, Dr.**
**Pfalzring 92**
**W-6704 Mutterstadt (DE)**
Erfinder : **Eisenbarth, Philipp, Dr.**
**Gutleutstrasse 12**
**W-6702 Bad Duerkheim (DE)**
Erfinder : **Allspach, Thomas, Dr.**
**Seebacher Strasse 58D**
**W-6702 Bad Duerkheim (DE)**

**EP 0 340 705 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein neues Polyphenol sowie daraus herstellbare Duromerharze.

Duromerharze, z.B. Epoxid-, Vinylester-, Bismaleinimid- oder Cyanatharze auf Basis üblicher Polyphenole, z.B. auf Bisphenol A-Basis, besitzen nach der Härtung gute mechanische Eigenschaften, jedoch ist ihre Hitzebeständigkeit für bestimmte Anwendungen nicht ausreichend. Werden Polyphenole, die durch Kondensation von Dicyclopentadien (DCPD) mit Phenol z.B. nach US 3 419 624 erhältlich sind, als Ausgangsstoffe für die oben genannten Duromerharze eingesetzt, so zeigen die entsprechenden gehärteten Polymeren zwar eine ausgezeichnete Hitzebeständigkeit, jedoch sind diese Materialien infolge der relativ hohen Vernetzungsdichte spröde.

Der Erfindung lag daher die Aufgabe zugrunde, neue Polyphenole zu entwickeln, aus denen sich Duromerharze, wie Epoxid-, Vinylester-, Bismaleinimid- oder Cyanatharze mit hoher Hitzebeständigkeit und guten mechanischen Eigenschaften herstellen lassen.

Diese Aufgabe wird durch die erfindungsgemäßen Polyphenolverbindungen gelöst.

Gegenstand der Erfindung ist eine Polypheholverbindung der Struktur

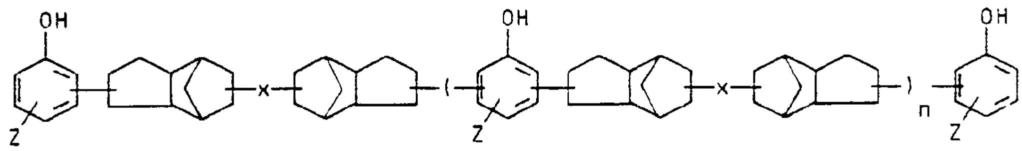 (I)

wobei n im Mittel zwischen 0 und 5 liegt, und

X = 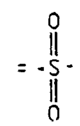 oder 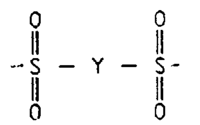 ist,

wobei Y eine Alkylen- oder Arylengruppen mit bis zu 15 C-Atomen ist, und Z=H, Hal, OH, Alkyl, Aryl, O-Alkyl, O- Aryl,

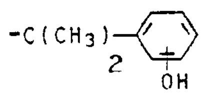

oder

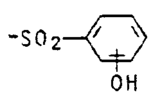

bedeutet.

Dabei ist in der allgemeinen Formel bevorzugt Z=H und X=$SO_2$ oder $SO_2$-$CH_2$-$CH_2$-$SO_2$; n liegt insbesondere zwischen 0 und 1.

1. Herstellung der Polyphenolverbindungen

Die Umsetzung von Schwefelwasserstoff mit Dicyclopentadien ist bekannt (DE-PS 1 259 880). Die Einsatzstoffe reagieren in Gegenwart eines radikalisch wirkenden Katalysators zu Bis-(dihydrodicyclopentadienyl)sulfid. Unter gleichen Bedingungen reagieren auch Dithiole. So erhält man z.B. aus Dithioglykol und DCPD Ethylen-bis-(dihydrodicyclopentadienyl)sulfid. Die Addition der Thiolgruppe erfolgt nur an die Doppelbindung im überbrückten Sechsring des DCPDs. Die Oxidation der Sulfide zu Sulfonen wird zweckmäßigerweise mit

Wasserstoffperoxid in Eisessig durchgeführt. Die verbleibende Doppelbindung im Fünfring des DCPDs wird in einer anschließenden Reaktion unter saurer Katalyse mit Phenol umgesetzt. Reaktionen von DCPD mit Phenolen werden in der Literatur beschrieben (z.B. J. Rivkin, W.E. Sheehan, Ind. Eng. Chem. 80 (1938) 1228; S.P. Panda, S.D. Kakade, J. Polym. Sci., Polym. Lett. Ed. 24 (1986) 403). Bei niedrigen Temperaturen (kinetische Reaktionskontrolle) werden Ether gebildet, bei höheren Temperaturen (thermodynamische Reaktionskontrolle) werden C-C-Bindungen geknüpft.

Außer Phenol können auch andere hydroxylhaltige aromatische Verbindungen eingesetzt werden, solange zumindest eine ortho- oder para-Ringposition in Bezug auf die Hydroxylgruppe frei ist. Als Beispiele seien genannt: Chlorphenol, Bromphenol, Methylphenol, Hydrochinon, Brenzkatechin, Resorcin, Pyrogallol, Isopropylphenol, Ethylphenol, Propylphenol, t-Butylphenol, Isobutylphenol, Phenylphenol, Bisphenol A, Dihydroxydiphenylsulfon.

2. Epoxidharze

Die Epoxidharze auf Basis der Polyphenolverbindungen I werden erhalten durch Umsetzung mit Epoxyalkylhalogeniden, vorzugsweisweise Epichlorhydrin.

Die Umsetzung der Epoxyalkylhalogenide mit der Polyphenolverbindung kann sowohl in einem Schritt als auch zweistufig unter Isolierung der Chlorhydrinetherzwischenstufe erfolgen. Es werden die üblichen Epoxidierungskatalysatoren, wie Ammonium-, Phosphonium- oder Arsoniumsalze eingesetzt. Die Dehydrohalogenierung führt man in Gegenwart von Basen, wie z.B. Kaliumcarbonat, wäßriger Natron- oder Kalilauge durch. Die Polyphenolverbindung kann gegebenenfalls teilweise, vorzugsweise zu weniger als 40 Mol-%, durch übliche Polyphenole, z.B. Bisphenol A, ersetzt sein.

Die erfindungsgemäßen Epoxidharze können direkt zur Herstellung von Formstoffen verwendet werden, beispielsweise von Formteilen, Faserverbundwerkstoffen, Beschichtungen und Klebstoffen. Dazu werden die Epoxidharze mit üblichen Härtern allein oder zusammen mit Härtungsbeschleunigern, in Mengen von 1 bis 150, vorzugsweise 3 bis 100 Gew.-Teilen, bezogen auf 100 Gew.-Teile Epoxidharz, versetzt und bei erhöhter Temperatur ausgehärtet. Geeignete Härter sind Polyamine, Polycarbonsäureanhydride und katalytische Härter, z.B. Hydrazide. Besonders bevorzugt sind Bis-(4-aminophenyl)methan, Bis-(4-aminophenyl)sulfon, Bis-(4-aminophenyl)-keton und Dicyandiamid. Den Epoxidharzen können übliche Zusatzstoffe, wie Verstärkungsfasern aus Glas oder Kohlenstoff, Füllstoffe, Kunststoffe und pulverförmige Metalle zugemischt werden. Die bei der Härtung erhaltenen Formstoffe zeichnen sich durch gute Chemikalienbeständigkeit und hohe Wärmeformbeständigkeit aus. Im Unterschied zu Epoxidharzen auf Basis von Bisphenol A oder zu den Harzen auf Basis der Dicyclopentadien-Phenol-Addukte nach US 4 390 680 haben die Epoxidharze auf Basis von der erfindungsgemäßen Polyphenolverbindung Vorteile hinsichtlich niedriger Wasseraufnahme und verbesserter Zähigkeit.

3. Vinylesterharze

Die Epoxidharze auf Basis der Polyphenolverbindung I können zur Herstellung entsprechender Vinylesterharze verwendet werden.

Dazu werden sie mit ungesättigten Monocarbonsäuren umgesetzt. Bevorzugte Monocarbonsäuren sind Acrylsäure, Methacrylsäure sowie Halbester ungesättigter Dicarbonsäuren, z.B. von Maleinsäure. Die Umsetzung wird ohne Katalysatoren oder in Gegenwart von Lewisbasen, wie z.B. tertiären Aminen, Triarylphosphinen, Acetaten, Alkoholaten oder Ammoniumhalogeniden bei 60 bis 130°C in einem inerten Lösungsmittel oder in der Schmelze durchgeführt. Dabei werden pro Epoxidgruppe vorzugsweise 0,6 bis 1,1 Äquivalente der ungesättigten Monocarbonsäure verwendet. Bei der Herstellung der Vinylester kann das erfindungsgemäße Epoxidharz teilweise, vorzugsweise zu weniger als 40 Mol%, durch übliche Epoxidharze, z.B. auf Basis von Bisphenol A oder von Novolaken, ersetzt werden.

Die Vinylesterharze enthalten

A. 25 bis 100 Gew.% der beschriebenen Vinylester,

B. 0 - 75 Gew.% eines ungesättigten, mit A copolymerisierbaren Monomeren, sowie

C. übliche Zusatzstoffe.

Als copolymerisierbare, ethylenisch ungesättigte monomere Verbindungen kommen die üblicherweise zum Herstellen ungesättigter Vinylesterharze verwendeten Allyl- und vorzugsweise Vinylverbindungen in Frage, z.B. Vinylaromaten, wie Styrol; substituierte Styrole, wie p-Chlorstyrol oder Vinyltoluol; Ester der Acrylsäure und Methacrylsäure mit 1 bis 18 Kohlenstoffatome enthaltenden Alkoholen, wie Methacrylsäuremethylester, Acrylsäurebutylester, Ethylhexylacrylat, Hydroxypropylacrylat, Dihydrodicyclopentadienylacrylat, Butandioldiacrylat und (Meth)-acrylsäureamide; Allylester, wie Diallylphthalat; ferner Vinylester wie Ethylhexansäurevinylester, Vinylacetat, Vinylpropionat, Vinylpivalat und andere. Desgleichen eignen sich Gemische der genannten olefinisch ungesättigten Monomeren. Bevorzugt geeignet als Komponente II sind Styrol, α-Methylstyrol, Chlorstyrol, Vinyltoluol, Divinylbenzol und Diallylphthalat.

Die erfindungsgemäß hergestellten Vinylesterharze können auch ohne Verdünnung mit einem ungesättigten Monomeren in Gegenwart von Polymerisationsinitiatoren ausgehärtet werden. Üblicherweise werden sie jedoch von 0 bis 75 Gew.%, vorzugsweise von 25 bis 75 Gew.%, mit einer copolymerisierbaren, ethylenisch ungesättigten Verbindung verdünnt. Auch sind Lösungen der monomerenfreien Vinylester in nicht polymerisierbaren Lösungsmitteln für die Herstellung von Prepregs sowie für Beschichtungszwecke möglich.

Übliche Polymerisationsinitiatoren sind Peroxide oder andere in der Wärme Radikale bildende organische Verbindungen in Mengen von 0,05 bis 5 Gew.%, vorzugweise von 0,1 bis 3 Gew.%, bezogen auf das Gesamtgewicht der Komponenten A + B. Als Radikale liefernde Initiatoren seien beispielhaft genannt: Benzoylperoxid, tert.-Butylperoctoat, tert.-Butylperbenzoat, Cyclohexanonperoxid, tert.-Dibutylperoxid und Hydroperoxide, ferner Azoverbindungen, wie Azodiisobutyronitril oder organische Verbindungen mit einer labilen Kohlenstoff-Kohlenstoff-Bindung. Bei Verwendung üblicher Lichtinitiatoren, wie z.B. Benzoinether, Benzilketalen oder Acylphosphinoxidverbindungen kann die Härtung durch Bestrahlen mit Licht der Wellenlänge 200 bis 500 nm durchgeführt werden.

Geeignete Füllstoffe sind z.B. übliche feinpulverige oder körnige anorganische Füllstoffe, wie Kreide, Kaolin, Quarzmehl, Dolomit, Schwerspat, Metallpulver, Zement, Talkum, Kieselgur, Holzmehl, Holzspäne, Pigmente und dergleichen. Als Verstärkungsfasern kommen solche aus Glas, Kohlenstoff oder aromatischem Polyamid in Frage. Sie werden in Mengen von 20 bis 200 Gew.%, bezogen auf A + B, eingesetzt.

Die erfindungsgemäßen Vinylesterharze werden bevorzugt zu faserverstärkten Formteilen ausgehärtet, die sowohl eine hohe Temperaturbeständigkeit als auch eine gute Zähigkeit aufweisen.

4. Bismaleinimid-Harze

Aus den Polyphenolverbindungen I können ferner durch Allylierung Polyallylphenol bzw. -ether II hergestellt werden, die als Härtungskomponente (Comonomere) für Bismaleinimide Verwendung finden.

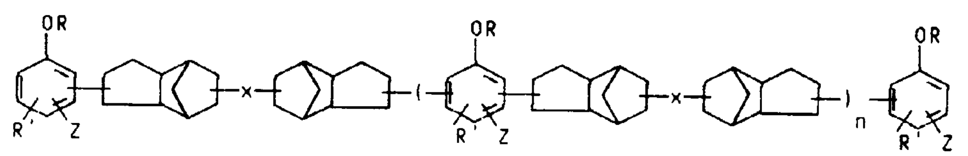

(II)

IIA: R = Allyl und R' = H
IIB: R = H und R' = Allyl

Die allylierten Derivate II werden durch Umsetzung der Polyphenole I mit gegebenenfalls substituierten Allylhalogeniden z.B. nach DE 2 818 091 erhalten. Geeignete Allylhalogenide sind Allylchlorid, Allylbromid, Methallylchlorid und Methallylbromid, wobei Allylchlorid bevorzugt ist. Dabei müssen die Substituenten Z gegenüber der Umsetzung mit Allylhalogeniden inert sein.

Man erhält dabei zunächst die entsprechenden O-Allylether IIA (R' = H, R = Allyl), die sich in einer Claisen-Umlagerung (nach DE 2 818 091) in die entsprechenden Allylphenole IIB (R' = Allyl, R = H) umwandeln lassen.

Bismaleinimidharze werden erhalten durch Umsetzung der Allylverbindungen II mit einem Bismaleinimid der allgemeinen Formel

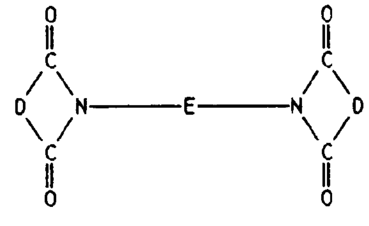
,

in der D eine gegebenenfalls substituierte Kohlenstoffdoppelbindung ist und E einen zweiwertigen Rest mit

zumindest zwei Kohlenstoffatomen bedeutet. Bismaleinimide sind z.B. aus DE-A-2 040 094, DE-A-2 719 903 und DE-A-3 247 058 bekannt. Neben Bismaleinimiden sind grundsätzlich auch Polymaleinimide, sowie Mischungen verschiedener Bismaleinimide geeignet. Bevorzugte Bismaleinimide sind 4,4'-Bismaleinimidodiphenylmethan, 4,4'-Bismaleinimidodiphenylether, 3,3'-Bismaleinimidodiphenylsulfon, 1,3-Bismaleinimidobenzol, 2,4-Bismaleinimidotoluol, 1,6-Bismaleinimidohexan und 2,2,4-Trimethyl-1,6-bismaleinimidohexan. Es können auch bis zu 20 Gew.% eines Monoimids enthalten sein.

Das Mischungsverhältnis bei der Umsetzung des Bismaleinimids mit der Allylverbindung ist relativ frei wählbar, wobei ein Äquivalent-Verhältnis von 1 zu 0,05 bis 5 vorzuziehen ist. Man nimmt an, daß bei der Umsetzung neben einer vermutlich radikalisch initiierten Copolymerisation auch Reaktionen vom Additionstyp, wie z.B. En-Reaktionen und Diels-Alder-Reaktionen ablaufen.

Je nach beabsichtigtem Verwendungszweck kann es vorteilhaft sein, den erfindungsgemäßen Harzen weitere Komponenten beizufügen. In Frage kommen beispielsweise übliche Epoxidharze oder Vinylesterharze.

Weitere einsetzbare Zusatzkomponenten sind Amine, bevorzugt aromatische Diamine (z.B. 4,4'-Diaminodiphenylmethan) und Aminophenole, die ebenfalls eine Additionsreaktion mit den Maleinimiddoppelbindungen eingehen können. Es können auch Präpolymere, z.B. aus einem Bisimid und einem Amin eingesetzt werden.

Für bestimmte Anwendungen kann es auch zweckmäßig sein, zur Einstellung einer gewünschten Viskosität geeignete Vinylmonomere, z.B. Styrol, $\alpha$-Methylstyrol, Divinylbenzol, Acryl- oder Methacrylsäureester, Diallylphthalat, 3,3'-Diallylbisphenol A, Triallylisocyanurat, Triallylcyanurat oder Vinylpyrrolidon einzusetzen. Ihr Anteil kann bis zu 50 Gew.%, bezogen auf die Mischung, betragen.

Die Mischungen können als weitere Zusätze Katalysatoren oder auch Inhibitoren enthalten. Geeignete Katalysatoren sind tertiäre Amine oder Phosphine, Imidazole oder organische Säuren oder Peroxide. Als Inhibitoren sind Hydrochinon, Benzochinon oder Phenothiazin zu nennen. Die Menge an eingesetzten Initiatoren und Inhibitoren soll etwa zwischen 0,05 - 1,5 Gew.% liegen.

Die Mischungen können weitere Zusätze, die in der Technologie der härtbaren Kunststoffe üblich sind, enthalten, wie Füllstoffe, Weichmacher, Pigmente, Farbstoffe, Formtrennmittel, flammhemmende Stoffe. Als Füllstoffe können auch Glas- und Kohlenstoffasern, Graphitpulver, Glimmer, Quarzpulver, Kaolin oder Metallpulver bis zu einem Anteil von 80 Gew.%, bezogen auf die Mischung, verwendet werden.

Die erfindungsgemäßen Mischungen sind als Imprägnier-, Gieß- und Laminierharze oder als Formmassen (gefüllt oder ungefüllt) einsetzbar.

Dienen sie zur Herstellung von Hochleistungsverbundwerkstoffen, so kann die Tränkung von Glas-, Kohlenstoff- oder Aramidfasern unter Bildung von unidirektionalen oder Gewebeprepregs entweder aus der Schmelze bei 50 - 150°C oder aus Lösung erfolgen. Geeignete Lösungsmittel sind halogenierte Kohlenwasserstoffe, wie z.B. Dichlormethan, Ketone wie z.B. Aceton oder Methylethylketon, Glykolester, Toluol, Dimethylformamid, N-Methylpyrrolidon bzw. Gemische aus mehreren Lösungsmitteln.

Zur Herstellung der Bismaleinimidharze werden die Ausgangsmaterialien unter Anwendung der üblichen Techniken vermischt und auf Temperaturen zwischen 70 - 190°C erhitzt, wobei die Bildung eines Präpolymeren erfolgt.

Je nach Fortschritt der Vorpolymerisation erhält man eine hochviskose Schmelze oder einen glasartig erstarrten Feststoff, der je nach Verwendungszweck gemahlen oder in einem Lösungsmittel gelöst wird. Die Herstellung der Harze kann auch in einem der oben genannten Lösungsmittel erfolgen.

Die Härtung der Harze erfolgt bei Temperaturen von ca. 100 bis 300°C, gegebenenfalls unter Druck, vorzugsweise zwischen 160 bis 260°C. Die gewählte Härtungstemperatur hängt entscheidend von der Länge der Härtungszeit ab und umgekehrt. Vielfach ist eine stufenweise Härtung vorteilhaft, wobei bei einer niedrigeren Temperatur zunächst unter Formgebung eine Vernetzung der Polymeren herbeigeführt wird. Nach Entformen kann sich dann zur vollständigen Aushärtung eine unter Umständen mehrstündige Nachhärtung oberhalb 200°C anschließen.

Aus den Harzen können Hochleistungswerkstoffe, z.B. Isoliermaterialien, Strukturbauteile, Apparategehäuse und elektrische Bauteile hergestellt werden, die hohen Temperaturen ausgesetzt sind.

5. Cyanatharze

Aus den Polyphenolverbindungen I können ferner durch Umsetzung mit Cyanhalogeniden neue Cyanatharze hergestellt werden. Man verfährt z.B. nach US 4 026 913, indem man zunächst Polyphenol und Bromcyan in einem Lösungsmittel, bevorzugt Aceton, vorlegt und durch Zugabe einer Base, z.B. Triethylamin, die Reaktion durchführt. Anstelle von Bromcyan kann auch Chlorcyan verwendet werden. Die so erhaltenen Cyanatharze lassen sich nach allgemein bekannten Methoden allein oder in Mischungen mit anderen Cyanatharzen, z.B. auf Basis von Bisphenol A, oder mit Epoxidharzen bzw. mit Bismaleinimiden zu hitzebeständigen

Duromeren härten. Als Härtungskatalysatoren kommen Metallverbindungen oder tertiäre Amine in Frage. Bevorzugt sind die Sn, Fe, Co, Ni, Cu, Zn-Salze organischer Säuren, z.B. die Acetylacetonate, Oktoate oder Naphthenate. Die Härtung erfolgt bei Temperaturen zwischen 150 - 250°C.

Die aus den Polyphenolen I herstellbaren Duromeren besitzen neben guten mechanischen Eigenschaften eine hervorragende Hitzebeständigkeit. Sie können zur Herstellung von Formstoffen verwendet werden, beispielsweise von Formteilen, Faserverbundwerkstoffen, Beschichtungen und Klebstoffen.

Beispiele

1. Herstellung eines Polyphenols

a) Bis-(dihydrodicyclopentadienyl)-sulfid

In einem 2 l-Vierhalskolben, der mit Rührer, Gaseinleitungsrohr, Thermometer und Rückflußkühler versehen ist, werden 1188 g DCPD und 23,8 g Azo-bis-isobutyronitril auf 60°C erhitzt. 168 g $H_2S$ werden über die Dauer von 10 Stunden eingeleitet. Dabei findet zunächst eine exotherme Reaktion statt, wobei die Temperatur 80 - 85°C nicht übersteigen soll. Nach 5 Stunden werden weitere 11,9 g Azobisisobutyronitril hinzugefügt. Nach Beendigung der $H_2S$-Einleitung wird 4 Stunden bei 85°C nachgerührt. Niedermolekulare Anteile werden bei 20 Torr entfernt, wobei die Temperatur bis 170°C angehoben wird. Es bleiben 1270 g einer gelben, viskosen Flüssigkeit zurück (91,2 % der Theorie).

b) Bis-(dihydrodicyclopentadienyl)-sulfon

In einem 6 l-Kolben werden 1030 g Bis-(dihydrodicyclopentadienyl)-sulfid und 2060 g Eisessig vorgelegt. Die Lösung wird auf 70 - 80°C erhitzt, und 494 g $H_2O_2$ (50 %ig) werden im Verlauf von 3,5 Stunden hinzugetropft. Anschließend wird 4 Stunden bei 75°C nachgerührt. Nach dem Abkühlen auf Raumtemperatur wird langsam $H_2O$ hinzugeführt, bis Kristallisation einsetzt (ca. 70 ml). Es wird auf 10°C abgekühlt, das Produkt wird abfiltriert, mit $H_2O$ gründlich gewaschen und bei 60°C im Vakuumtrockenschrank getrocknet. Es bleiben 810 g eines hellgelben Pulvers zurück (71 % der Theorie).

c) Polyphenol auf Basis Bis-(dihydrodicyclopentadienyl)-sulfon

760 g Bis-(dihydrodicyclopentadienyl)-sulfon, 1600 g Phenol und 575 g Lewatit SPC 118H+ (Fa. Bayer, saurer Ionenaustauscher) werden in einem 6 l-Kolben langsam auf 110°C erhitzt und 24 Stunden bei dieser Temperatur gerührt. Es wird auf 80°C abgekühlt, der Ionenaustauscher wird abfiltriert und mit Phenol gewaschen. Mittels Vakuumdestillation (200°C, 1 mbar) werden Phenol und sonstige flüchtige Anteile entfernt.

| | |
|---|---|
| Auswaage : | 850 g (71 % der Theorie) |
| Aussehen : | dunkelrot - violett |
| Erweichungspunkt (Kofler): | 125°C |
| Elementaranalyse: | |

| | | theoretische Werte bei bifunktioneller Verbindung |
|---|---|---|
| C | 72,2 % | 74,1 |
| H | 7,9 % | 7,4 |
| O | 11,0 % | 12,3 |
| S | 8,0 % | 6,2 |

2. Herstellung eines Epoxidharzes

400 g der Polyphenolverbindung 1c werden in 2150 g Epichlorhydrin zu leichtem Rückfluß erwärmt. Anschließend werden 106 g 50 %ige Natronlauge zudosiert. Das Azeotrop aus Epichlorhydrin und Wasser wird dabei kontinuierlich aus dem Reaktionsgefäß herausdestilliert, so daß die Wasserkonzentration bei etwa 1 %

gehalten wird. Nach Ende des Laugenzulaufs (2 Stunden) wird noch 30 Minuten nachreagiert, anschließend auf 80°C abgekühlt und mit 500 ml Wasser versetzt. Nach erfolgter Phasentrennung wird die organische Schicht noch zweimal mit Wasser gewaschen und anschließend über einen Dünnschichtverdampfer vom überschüssigen Epichlorhydrin befreit (170°C, 3 mbar).

Das rotbraun gefärbte Epoxidharz weist einen Erweichungspunkt von 66°C und ein Epoxidäquivalentgewicht von 370 auf.

3. Herstellung eines Vinylesterharzes

370 g des Epoxidharzes aus Beispiel 2 werden mit 0,2 g Hydrochinonmonomethylether und 0,5 g Benzyltriethylammoniumchlorid versetzt und auf 120°C erwärmt. Innerhalb von 10 Minuten werden 86 g Methacrylsäure zugegeben und 6 Stunden auf 105 - 110°C erwärmt. Dabei setzen sich ca. 95 % aller Epoxidgruppen um, der Reaktionsverlauf wird durch Messen der Säurezahl verfolgt. Der entstandene Vinylester wird bei 100°C in 304 g Styrol gelöst. Die entstandene Lösung besitzt bei Raumtemperatur eine Viskosität von 450 mPas.

In die im vorstehenden Abschnitt beschriebene styrolische Harzlösung werden bei 23°C 2,0 % Methylethylketonperoxid und 1 % Cobaltnaphthenat (1 %ig in Styrol) eingerührt und zu einer 4 mm dicken Reinharzplatte gehärtet (2 Stunden Raumtemperatur, 20 Stunden 160°C).

Folgende Eigenschaften wurden gemessen:

| | |
|---|---|
| Zug-E-Modul | : 3600 N/mm$^2$ |
| Zugfestigkeit | : 57 N/mm$^2$ |
| Reißdehnung | : 3,2 % |
| Glastemperatur | : 156°C |

4. Herstellung eines Bismaleinimidharzes

a) Allylierung des Polyphenols 1c

Die Mischung aus 158 g Polyphenol nach 1c), 22 g Natriumhydroxid und 1000 ml n-Propanol wird auf ca. 90°C aufgeheizt und innerhalb 1 Stunde werden 45 ml Allylchlorid zugetropft. Man rührt weitere 10 Stunden unter Rückfluß und filtriert nach Abkühlen das ausgefallene Natriumchlorid ab. Nach Abdestillieren des Lösungsmittels verbleiben 170 g des entsprechenden O-Allylethers als hochviskoses Öl mit einer Viskosität von 2500 mPas bei 100°C.

b) Claisen-Umlagerung des Allylethers

155 g des Allylethers nach 4a) werden nach Anlegen eines Vakuums von ca. 1 mbar 3 Stunden auf 200°C erhitzt. Man erhält 149 g Allylphenol als rotbraunes hochviskoses Harz, das bei Raumtemperatur erstarrt;
Erweichungspunkt: ca. 65°C (Koflerbank);
Viskosität bei 125°C: 3200 mPas.

c) Herstellung eines Bismaleinimidharzes aus Allylphenol 4b)

In einem Reaktionsgefäß werden unter Rühren bei einer Badtemperatur von 160°C zu 120 g Allylphenol nach b) sowie 0,8 g 2,6-Dimethylhydrochinon 280 g 4,4'-Bismaleinimidodiphenylmethan portionsweise zugegeben. Die niedrigviskose Harzmasse wird nach 10minütigem Erhitzen wie folgt weiterverarbeitet:

1. Ein kleiner Teil des Harzes wird zwecks schnelleren Abkühlens auf eine Metallfolie gegossen. Nach Erkalten besitzt das rotbraune Harz einen Erweichungspunkt von 50°C (Koflerbank); seine Gelzeit bei 160°C beträgt 38 Minuten.
2. Der restliche Teil der Harzmasse wird in 1 mm und 4 mm dicke Metallformen gegossen und 2 Stunden bei 160°C, 5 Stunden bei 190°C und 8 Stunden bei 240°C gehärtet. Das Polymer besitzt eine Glasübergangstemperatur oberhalb von 300°C (nach DIN 53455); bei 325°C besitzt es noch 50 % seines Raumtemperatur-Schubmoduls (nach DIN 53455); E-Modul: 3990 N/mm$^2$ (DIN 53457).

5. Herstellung eines Cyanatharzes

Zur Lösung von 143,6 g Polyphenol nach Beispiel 1c), 100 ml einer 5 molaren Lösung von Bromcyan in Acetonitril in 1000 ml Aceton werden bei 0°C innerhalb 30 Minuten 55,2 g Triethylamin zugetropft. Man rührt noch 2 Stunden bei 0°C nach und filtriert nach Auftauen das ausgefallene Triethylammoniumbromid ab. Die

Mutterlauge wird im Vakuum eingedampft, und der Rückstand wird in 2 l Dichlormethan gelöst. Anschließend wird mit viel Wasser gewaschen, über Natriumsulfat getrocknet und im Vakuum bei max. 30°C eingedampft. Man erhält 104 g (67 %) Cyanatharz mit einem Erweichungspunkt von 45°C (Koflerbank); IR (KBr): 2240, 2260 $cm^{-1}$ (-OCN).

Nach Zugabe von 0,05 % Kobalt-naphthenat und 2-stündigem Härten bei 170 sowie 12-stündigem Härten bei 220°C entsteht ein Polymer mit einer Glasübergangstemperatur von 224°C.

## Patentansprüche

1. Polyphenolverbindung der Struktur

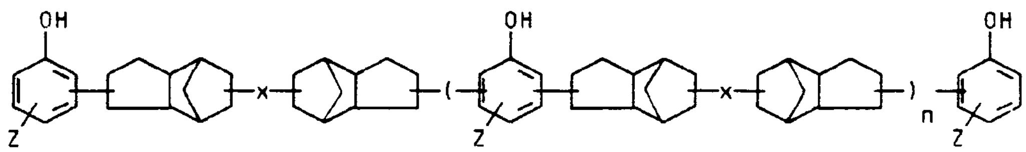
(I)

wobei n im Mittel zwischen 0 und 5 liegt, und

X = 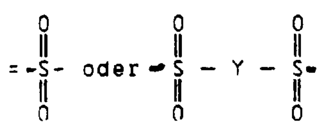 oder ist,

wobei Y eine Alkylen- oder Arylengruppen mit bis zu 15 C-Atomen ist, und Z=H, Hal, OH, Alkyl, Aryl, O-Alkyl, O-Aryl,

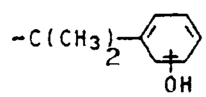

oder

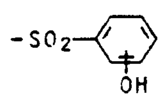

bedeutet.

2. Epoxidharze auf Basis der Polyphenolverbindung nach Anspruch 1.

3. Vinylesterharze auf Basis der Polyphenolverbindung nach Anspruch 1.

4. Bismaleinimidharze, enthaltend ein allyliertes Derivat der Polyphenolverbindung nach Anspruch 1 als Comonomer.

5. Cyanatharze auf Basis der Polyphenolverbindung nach Anspruch 1.

## Claims

1. A polyphenol compound having the structure

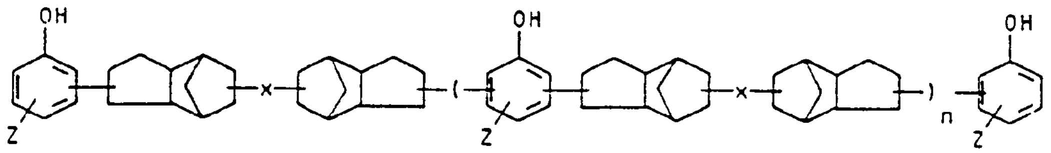 (I)

where n is on average from 0 to 5 and

X is 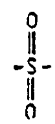 or 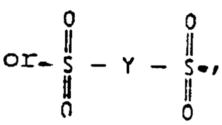,

Y is an alkylene or arylene group of not more than 15 carbon atoms and Z is H, Hal, OH, alkyl, aryl, O-alkyl, O-aryl,

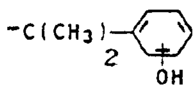 or 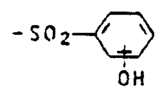 .

2. An epoxy resin based on a polyphenol compound as claimed in claim 1.

3. A vinyl ester resin based on a polyphenol compound as claimed in claim 1.

4. A bismaleimide resin containing an allylated derivative of a polyphenol compound as claimed in claim 1 as comonomer.

5. A cyanate resin based on a polyphenol compound as claimed in claim 1.

## Revendications

1. Dérivé de polyphénol de formule

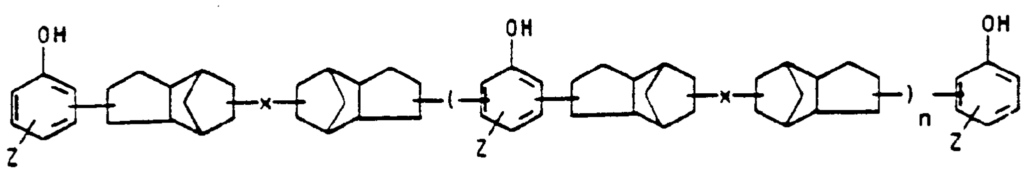 (I)

dans laquelle n est compris en moyenne entre 0 et 5, et

X est 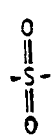 ou 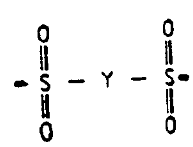

où Y est un groupement alkylène ou arylène ayant jusqu'à 15 atomes de carbone, et Z est H, Hal, OH, un groupement alkyle, aryle, O-alkyle, O-aryle,

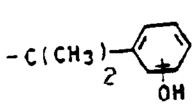 ou 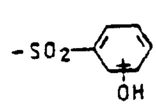 .

2. Résines époxy à base du dérivé de polyphénol selon la revendication 1.

3. Résines d'esters vinyliques à base du dérivé de polyphénol selon la revendication 1.

4. Résines de bismaléimide, contenant un dérivé allylé du dérivé de polyphénol selon la revendicatian 1 comme comonomère.

5. Résines de cyanate à base du dérivé de polyphénol selon la revendication 1.